# EUROPÄISCHE PATENTSCHRIFT

(11) **EP 0 772 576 B1**
(45) Veröffentlichungstag und Bekanntmachung des Hinweises auf die Patenterteilung: **20.01.1999**
(21) Anmeldenummer: 95926901.0
(22) Anmeldetag: 15.07.1995
(51) Int. Cl.: C07C 17/02, C07C 19/045

(54) **VERFAHREN UND VORRICHTUNG ZUR HERSTELLUNG VON 1,2-DICHLORETHAN DURCH DIREKTCHLORIERUNG UNTER ABGASRÜCKFÜHRUNG**
PROCESS AND DEVICE FOR PREPARING 1,2-DICHLOROETHANE BY DIRECT CHLORINATION WITH WASTE GAS RECIRCULATION
PROCEDE ET DISPOSITIF DE PRODUCTION DE 1,2-DICHLOROETHANE PAR CHLORATION DIRECTE AVEC RECYCLAGE DES EFFLUENTS GAZEUX

(30) Priorität: 21.07.1994 DE 4425872
(43) Veröffentlichungstag der Anmeldung: 14.05.1997
(73) Patentinhaber: Vinnolit Monomer GmbH & Co. KG, 85737 Ismaning (DE)
(72) Erfinder: EICHLER, Jürgen, D-84556 Kastl (DE); MIELKE, Ingolf, D-84508 Burgkirchen (DE); SCHWARZMAIER, Peter, D-84556 Kastl (DE); STÖGER, Manfred, D-84508 Burgkirchen (DE); WILD, Thomas, D-84508 Burgkirchen (DE)
(74) Vertreter: Schuderer, Michael, Dr.
(86) Internationale Anmeldenummer: EP9502787
(87) Internationale Veröffentlichungsnummer: WO9603361

(56) Entgegenhaltungen:
- EP-A- 0 075 742
- DE-A- 4 318 609

## Beschreibung

Die Erfindung betrifft ein Verfahren zur Herstellung von 1,2-Dichlorethan, im folgenden "EDC", durch Einspeisung von Chlor und Ethylen in im Kreislauf geführtes EDC, das ein Katalysatorsystem aus Eisen-(III)-chlorid und einem Halogenid eines Metalls der ersten oder zweiten Hauptgruppe des periodischen Systems der Elemente sowie geringe Mengen Sauerstoff enthält, wobei man einen Teilstrom des Reaktionsgemisches einer Entspannungsverdampfung zuführt, das verdampfte EDC von leichter flüchtigen Bestandteilen abtrennt und isoliert, das dadurch gekennzeichnet ist, daß man die genannten leichter flüchtigen Bestandteile in die Reaktion zurückfördert, wobei die Kombination der folgenden Verfahrensmerkmale ausgenommen ist: Das Metallchlorid im Katalysatorsystem ist Natriumchlorid, wobei während der gesamten Umsetzung das Molverhältnis von Natriumchlorid zu Eisen-(III)-chlorid unter 0,5 bleibt, die Entspannungsverdampfung erfolgt in einem unter Unterdruck stehenden Behälter und ethylenhaltiges Abgas wird mit einem Verdichter in die Reaktion zurückgeführt.

Das Verfahren zur Herstellung von EDC durch Umsetzung von Chlor und Ethylen ist unter dem Begriff "Direktchlorierung" bekannt. So betrifft zum Beispiel die DE-A 41 33 810 ein solches Verfahren, bei dem mit Hilfe der Entspannungsverdampfung die Reaktionsenthalpie genutzt wird. Dieses bekannte Verfahren wird bei einer Temperatur von etwa 75 bis 200 °C und einem Druck von etwa 1 bis 15 bar durchgeführt.

Es wurde auch schon ein Verfahren zur Herstellung von EDC durch Umsetzung von Ethylen mit Chlor an einem Natriumchlorid-Eisen-(III)-chlorid-Katalysator vorgeschlagen, das dadurch gekennzeichnet ist, daß während der gesamten Umsetzung das Molverhältnis von Natriumchlorid zu Eisen-(III)-chlorid unter 0,5 bleibt. Dieses Verfahren wird vorzugsweise bei 50 bis 105 °C und einem Druck von bis zu 1 bar Überdruck durchgeführt. In einer weiteren bevorzugten Ausführungsform wird das EDC ganz oder teilweise in einen unter Unterdruck stehenden Behälter geleitet, wobei das abdestillierende Produkt abgetrennt und das restliche EDC in den Prozeß zurückgeführt wird. Als zweckmäßige Ausgestaltung wird weiterhin vorgeschlagen, zur Verminderung der Ethylenverluste das ethylenhaltige Abgas aus dem Entspannungsbehälter nach der Vakuumpumpe (die für die Aufrechterhaltung des Unterdrucks im Entspannungsbehälter erforderlich ist) mittels eines geeigneten Verdichters, bevorzugt eines Flüssigkeitsstrahlverdichters, der zweckmäßig am Boden des Reaktors angebracht ist, in die Reaktion zurückzuführen und das Ethylen erneut mit Chlor umzusetzen. Hierbei dient als Treibstrahl für den bevorzugten Verdichter vorteilhaft ein Teilstrom des umgepumpten EDC (deutsche Patentanmeldung P 43 18 609.2 vom 04.06.1993).

Das erfindungsgemäße Verfahren ist außerordentlich vielseitig: Die Umsetzung kann in einem Temperaturbereich von etwa 50 bis 200 °C und bei einem Druck von etwa 1 bis etwa 15 bar Überdruck erfolgen. Der Druck im Entspannungsbehälter muß entsprechend niedriger sein und liegt im Bereich von 0,2 bis 3,5 bar absolut. In einer Ausführungsform des Verfahrens beträgt dieser Druck im Entspannungsbehälter mehr als 0,7 bar absolut.

Das Metallhalogenid im Katalysatorsystem ist vorzugsweise Lithiumchlorid, Kaliumchlorid oder Magnesiumchlorid und insbesondere Natriumchlorid. Das Molverhältnis von Metallhalogenid und Eisen-(III)-chlorid kann in gewissen Grenzen schwanken und liegt bei den bekannten Verfahren zwischen 0,5 : 1 und 2 : 1 (NL-A 6901398, DE-A 41 03 281).

Das eingesetzte Chlor kann flüssig oder gasförmig zugeführt werden. Es ist auch nicht erforderlich, Chlor der höchsten Qualitätsstufe einzusetzen: Durch die geeignete Wahl eines Verdichters, beispielsweise durch den in dem vorgeschlagenen Verfahren genannten Flüssigkeitsstrahlverdichter, kann ein technisches Chlor eingesetzt werden, das geringe Anteile Sauerstoff enthält. Eine andere Möglichkeit ist der Einsatz eines entsprechend explosions- beziehungsweise druckstoßsicheren Verdichters anderer Bauart.

Bei Wahl des Flüssigkeitsstrahlverdichters dient auch beim erfindungsgemäßen Verfahren bevorzugt ein Teilstrom des umgepumpten EDC, insbesondere ein Teilstrom des in den Reaktor zurückgeführten und vorzugsweise gekühlten EDC, als Treibstrahl.

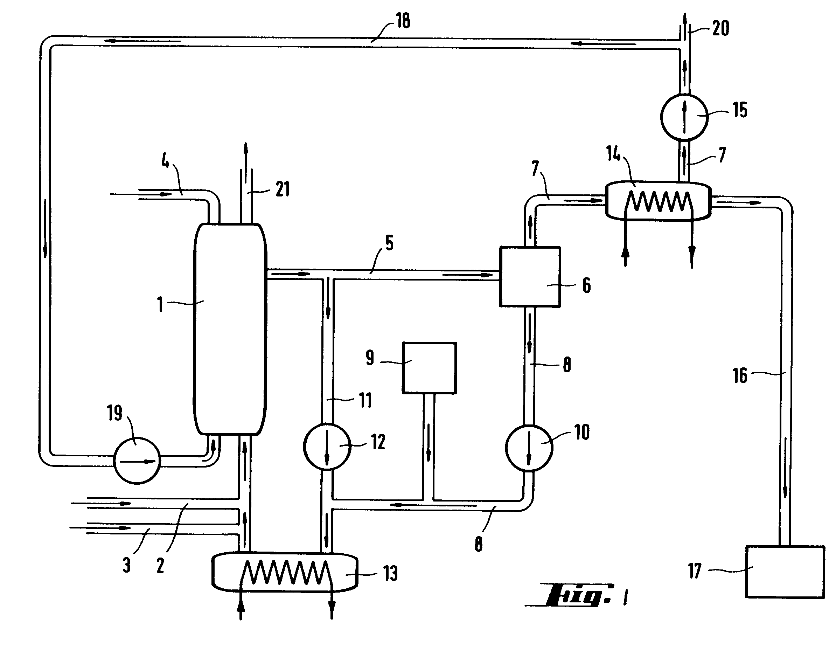

Das erfindungsgemäße Verfahren wird vorteilhaft in einer Apparatur entsprechend der Figur durchgeführt. Diese Vorrichtung enthält
einen Reaktor (1),
Zuführungen (2) und (3) für Ethylen beziehungsweise Chlor sowie vorzugsweise (4) für ein Inertgas,
eine Leitung (5) zum
Entspannungsbehälter (6), der unter unterdruck steht und eine Leitung (7) zur Ableitung des verdampften Produkts und
eine Leitung (8) zur Rückführung des unverdampften Produkts hat,
an die vorzugsweise ein Lösebehälter (9) angeschlossen ist und die
vorzugsweise über eine Pumpe (10) führt,
eine Leitung (11) zur Rückführung eines
Produktteilstroms,
die vorzugsweise über eine Pumpe (12) und einen Kühler beziehungsweise wärmetauscher (13) führt.

Die Leitung (7) für den EDC-Dampf führt zweckmäßig über einen Kühler (14) zur Vakuumpumpe (15). Vom Kühler (14) führt die Leitung (16) zum Sammelbehälter (17) für das verflüssigte EDC.

Die Vorrichtung enthält weiterhin eine Leitung (18) von der (Druckseite der) Vakuumpumpe (15) über den Verdichter (19) zum Reaktor (1) zur Rückführung von Restmengen unumgesetzten Ethylens. (20) und (21) sind Leitungen zur Abgasentsorgung.

Die Erfindung wird in dem folgenden Beispiel näher erläutert.

### Beispiel

Zur Direktchlorierung wurde ein Reaktor (1) mit einem Volumen von 14,6 m³ mit 3000 Nm³/h Ethylen über die Leitung (2) beschickt. Über die Leitung (3) wurde der äquivalente Chlorstrom eingespeist. Der Druck wurde über die Leitung (4) mit Stickstoff auf 0,8 bar Überdruck und die Temperatur bei 90 °C gehalten. Als Reaktionsmedium diente flüssiges EDC mit einem Eisen-(III)-chlorid-Gehalt von 780 ppm.

In das Reaktionsmedium wurde über den Lösebehälter (9) soviel Natriumchlorid eindosiert, bis die gewünschte Konzentration von circa 170 ppm erreicht war und aufrechterhalten blieb.

Ein Produktteilstrom wurde über die Leitung (5) in den nachgeschalteten Entspannungsbehälter (6) mit einem Druck von 0,7 bis 0,8 bar absolut geführt. Hier wurden pro Stunde 13,3 t EDC verdampft und über die Leitung (7) abgezogen. Dieses EDC war katalysatorfrei und wies eine Reinheit von 99,92 % auf.

Das im Entspannungsbehälter (6) nicht verdampfte EDC wurde über die Leitung (8) und die Pumpe (10) in den Reaktor (1) zurückgeführt. Der Produkthauptstrom wurde über die Leitung (11) und die Pumpe (12) in die Leitung (8) eingespeist und über den Kühler (13) in den Reaktor (1) zurückgeführt.

Über die Abgasleitung (21) wurden stündlich 50 Nm³/h mit 6 Vol.-% Ethylengehalt der Verbrennung zugeführt. Dies entspricht 3 Nm³/h Ethylenverlust. Die Abgasleitung (20) war geschlossen.

Wird demgegenüber über die Abgasleitung (21) ein Strom von 28 Nm³/h mit 6 Vol.-% Ethylengehalt und über die Abgasleitung (20) ein Strom von 26 Nm³/h mit 16 Vol.-% Ethylen der Verbrennung zugeführt, so entspricht das insgesamt einem Ethylenverlust von 5,9 Nm³/h oder 2,9 Nm³/h mehr als nach der erfindungsgemäßen Betriebsweise. Das erfindungsgemäße Verfahren mit geschlossener Abgasleitung (20) und Rückführung des Gasstroms über die Leitung (18) halbiert somit praktisch den Ethylenverlust auf etwa 0,1 % der eingesetzten Ethylenmenge.

## Patentansprüche

1. Verfahren zur Herstellung von 1,2-Dichlorethan (EDC) durch Einspeisung von Chlor und Ethylen in im Kreislauf geführtes EDC, das ein Katalysatorsystem aus Eisen-(III)-chlorid und einem Halogenid eines Metalls der ersten oder zweiten Hauptgruppe des periodischen Systems der Elemente sowie geringe Sauerstoffmengen enthält, wobei man einen Teilstrom des Reaktionsgemisches einer Entspannungsverdampfung zuführt, das verdampfte EDC von leichter flüchtigen Bestandteilen abtrennt und isoliert, dadurch gekennzeichnet, daß man die genannten leichter flüchtigen Bestandteile in die Reaktion zurückfördert, wobei die Kombination der folgenden Verfahrensmerkmale ausgenommen ist: Das Metallchlorid im Katalysatorsystem ist Natriumchlorid, wobei während der gesamten Umsetzung das Molverhältnis von Natriumchlorid zu Eisen-(III)-chlorid unter 0,5 bleibt, die Entspannungsverdampfung erfolgt in einem unter Vakuum stehenden Behälter und ethylenhaltiges Abgas wird mit einem Verdichter in die Reaktion zurückgeführt.

2. Verfahren nach Anspruch 1, dadurch gekennzeichnet, daß die Reaktion bei einer Temperatur von etwa 50 bis 200 °C und einem Druck von etwa 1 bis etwa 15 bar Überdruck erfolgt.

3. Verfahren nach Anspruch 2, dadurch gekennzeichnet, daß die Entspannungsverdampfung bei einem Druck von 0,2 bis 3,5 bar absolut erfolgt.

## Claims

1. Process for preparing 1,2-dichloroethane (EDC) by feeding chlorine and ethylene into circulated EDC which contains a catalyst system of iron(III) chloride and a halide of a metal of the first or second main group of the Periodic Table of the Elements and small amounts of oxygen, a part-stream of the reaction mixture being fed to flash evaporation, the vaporized EDC being separated off from more readily volatile constituents and isolated, characterized in that the said more readily volatile constituents are returned to the reaction, the combination of the following process features being excepted: the metal chloride in the catalyst system is sodium chloride, the molar ratio of sodium chloride to iron(III) chloride remaining below 0.5 during the entire reaction, the flash evaporation proceeds in a vessel under reduced pressure and ethylene-containing off-gas is recycled to the reaction by a compressor.

2. Process according to Claim 1, characterized in that the reaction proceeds at a temperature of about 50 to 200°C and at a pressure of about 1 to about 15 bar gauge pressure.

3. Process according to Claim 2, characterized in that the flash evaporation proceeds at a pressure of 0.2 to 3.5 bar absolute.

## Revendications

1. Procédé de préparation du 1,2-dichloroéthane (EDC), comprenant l'addition de chlore et d'éthylène dans de l'EDC mis en circulation qui contient un système catalytique de chlorure de fer (III) et un halogénure de métal du premier ou second groupe principal du tableau périodique des éléments et des petites quantités d'oxygène, un flux partiel du mélange de réaction étant alimenté vers une évaporation éclair, l'EDC vaporisé étant séparé des constituants plus facilement volatils et isolé, caractérisé en ce que l'on revendique lesdits constituants plus facilement volatils vers la réaction, la combinaison des particularités suivantes du procédé étant exclue: le chlorure métallique dans le système catalytique est le chlorure de sodium, le rapport molaire chlorure de sodium/chlorure de fer (III) restant inférieur à 0,5 pendant toute la réaction, l'évaporation éclair s'effectue dans une cuve sous pression réduite, et le gaz sortant contenant de l'éthylène est recyclé vers la réaction par un compresseur.

2. Procédé suivant la revendication 1, caractérisé en ce que la réaction s'effectue à une température d'environ 50 à 200°C et sous une pression d'environ 1 à environ 15 bars manométriques.

3. Procédé suivant la revendication 2, caractérisé en ce que l'évaporation éclair s'effectue sous une pression de 0,2 à 3,5 bars absolus.
